# EUROPEAN PATENT APPLICATION

(11) **EP 4 825 365 A1**
(43) Date of publication of application: **21.10.2026**
(21) Application number: 24903327.5
(22) Date of filing: 17.10.2024
(51) Int. Cl.: A61K 8/31, A61K 8/19, A61K 8/34, A61K 8/73, A61K 8/365, A61Q 11/00

(54) **NONAQUEOUS COMPOSITION FOR ORAL CAVITY**

(30) Priority: 15.12.2023 JP 2023211835
(71) Applicant: Kao Corporation, Tokyo 103-8210 (JP)
(72) Inventor: TOI, Manabu, Tokyo 103-8210 (JP); OYAMA, Tsutomu, Tokyo 103-8210 (JP); SATO, Takaki, Tokyo 103-8210 (JP); NAGATA, Ryosuke, Tokyo 103-8210 (JP); YAMAMOTO, Masaya, Tokyo 103-8210 (JP)
(74) Representative: Hoffmann Eitle
(86) International application number: PCT/JP2024/036995
(87) International publication number: WO 2025/126664

(57) **Abstract**

The present invention relates to a nonaqueous composition for oral cavity capable of providing a pleasant fizzy feeling and a good use impression with reduced oily feeling when applied to the oral cavity, and also effectively prevents generation of carbon dioxide gas after storage. That is, the present invention provides a nonaqueous composition for oral cavity containing the following components (A) to (E): (A) a nonaqueous solvent having a relative permittivity at 25°C of less than 10 and a melting point of 0°C or lower; (B) an acid which is solid at 25°C; (C) a carbonate salt; (D) an oil-based gelling agent; and (E) a sugar alcohol which is solid at 25°C.

## Description

### Field of the Invention

The present invention relates to a nonaqueous composition for oral cavity.

### Background of the Invention

A nonaqueous composition for oral cavity containing an organic acid and a carbonate salt as a carbon dioxide gas generating agent can generate carbon dioxide gas when it is applied to the oral cavity and produce a characteristic fizzy feeling. Thus, various nonaqueous compositions for oral cavity have been conventionally developed.

For example, Patent Literature 1 discloses a composition for oral cavity containing a base for a nonaqueous composition for oral cavity, sodium bicarbonate, and an acidic component which is solid at room temperature, and describes attempts to enhance the fizzy characteristics while reducing bitterness, astringency, and the like. In addition, Patent Literature 2 discloses a pasty dentifrice containing an organic acid coated with a coating agent, sodium bicarbonate coated with a coating material, and polyacrylic acid, and contemplates the suppression of carbon dioxide gas during long-term storage.

(Patent Literature 1) JP 2002-302429 A
(Patent Literature 2) JP 2022-81908 A

### Summary of the Invention

The present invention provides a nonaqueous composition for oral cavity, comprising the following components (A) to (E):
(A) a nonaqueous solvent having a relative permittivity at 25°C of less than 10 and a melting point of 0°C or lower;
(B) an acid which is solid at 25°C;
(C) a carbonate salt;
(D) an oil-based gelling agent; and
(E) a sugar alcohol which is solid at 25°C.

Even with the technologies described in any of the patent literatures described above, there is still room for improvement in achieving a pleasant fizzy feeling and a good use impression with a reduced oily feeling in the oral cavity where the composition is applied, while also effectively preventing the generation of the carbon dioxide gas after the storage.

Accordingly, the present invention relates to a nonaqueous composition for oral cavity which is capable of producing a pleasant fizzy feeling and a good use impression with a reduced oily feeling when applied to the oral cavity, and capable of effectively preventing the generation of the carbon dioxide gas even after the storage.

The present inventors found, as a result of various studies, that a nonaqueous composition for oral cavity containing a nonaqueous solvent having a relative permittivity of a specific value, an acid which is solid at 25°C, a carbonate salt, an oil-based gelling agent, and a sugar alcohol which is solid at 25°C retains good manufacturing stability and provides a good fizzy feeling and use impression upon application, and also exhibits excellent storage stability, thereby effectively preventing the generation of the carbon dioxide gas even after the storage.

According to the nonaqueous composition for oral cavity of the present invention, when it is applied to the oral cavity, the carbon dioxide gas forms bubbles which produces a characteristic and pleasant fizzy feeling and pleasantly burst in the oral cavity, while suppressing an unpleasant oily feeling, resulting in a refreshing feeling after the use. Further, the nonaqueous composition for oral cavity of the present invention possesses good shape retention property and manufacturing stability while exhibiting excellent storage stability, effectively preventing the generation of the carbon dioxide gas after the storage, and avoiding, during the storage, undesirable swelling of a container in which the composition is filled.

### Brief Description of the Drawing

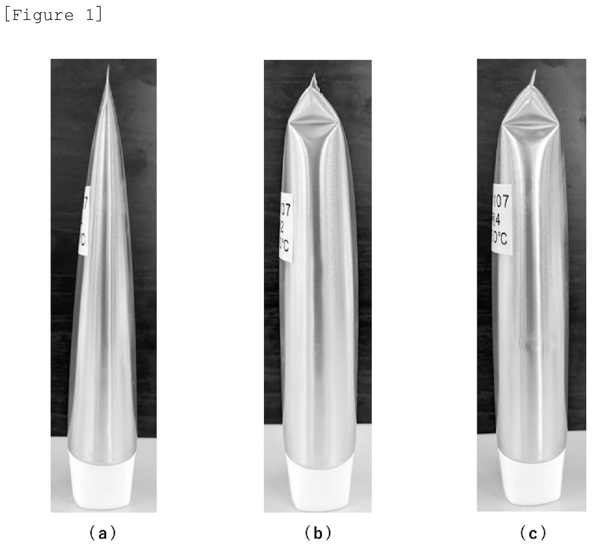

Figure 1 illustrates photographs showing lateral views of a container standing upright after storage in evaluation of the storage stability. Figure 1(a) illustrates a photograph of Example 1, Figure 1(b) illustrates a photograph of Comparative Example 1, and Figure 1(c) illustrates a photograph of Comparative Example 2.

### Detailed Description of the Invention

The present invention will be described in detail below. As used herein, the term "nonaqueous composition for oral cavity" means a composition for oral cavity that is a composition containing no water as a contained component, and substantially does not contain water, except for the case where water is inevitably mixed into the composition. The phrase "substantially does not contain water" means that the moisture content of the composition for oral cavity is preferably less than 2% by mass, more preferably less than 1% by mass.

Further, the term "good fizzy feeling" means that, when the nonaqueous composition for oral cavity of the present invention is applied to the oral cavity, an acid which is solid at 25°C and a carbonate salt, which are contained as a carbon dioxide gas generating agent in the nonaqueous composition for oral cavity of the present invention, start a reaction due to the moisture in the saliva to generate the carbon dioxide gas, and the carbon dioxide gas forms bubbles which spread throughout the oral cavity while pleasantly bursting with a characteristic and pleasant fizzy feeling, thereby providing a refreshing feeling.

Furthermore, the term "good use impression" means a sensation in which, when the composition is applied to the oral cavity, an unpleasant oily feeling is also suppressed and the flavor is not impaired.

Accordingly, the term "refreshing feeling" provided by the nonaqueous composition for oral cavity of the present invention is used as a generic term for an excellent sensation in the oral cavity, which includes not only a good "fizzy feeling" and "use impression" when it is applied to the oral cavity but also a "fresh feeling" which is perceived even after the application.

The nonaqueous composition for oral cavity of the present invention contains, as a component (A), a nonaqueous solvent having a relative permittivity at 25°C of less than 10 and a melting point of 0°C or lower. In the nonaqueous composition for oral cavity of the present invention, while it contains, as a carbon dioxide gas generating agent, an acid which is solid at 25°C as a component (B) and a carbonate salt as a component (C) (described later), the presence of the component (A) can effectively prevent undesired generation of the carbon dioxide gas during the manufacturing, during the storage, or before the use, thereby contributing to the ensuring of the good storage stability while maintaining the excellent manufacturing stability. Further, when the nonaqueous composition for oral cavity of the present invention is applied to the oral cavity, it contacts with the saliva and the like, which promotes the reaction between the component (B) and the component (C), so that bubbles of the carbon dioxide gas, which exhibit a characteristic and pleasant fizzy feeling in the oral cavity, are generated and spread throughout the oral cavity, while pleasantly bursting, thereby providing a refreshing feeling.

It should be noted that, as used herein, the term "nonaqueous solvent" refers to a solvent other than water, which substantially does not contain water. Further, the term "melting point" refers to a value at 1 atm.

The relative permittivity at 25°C of the component (A) is less than 10, and is preferably 5 or less, more preferably 3 or less. The melting point of the component (A) is 0°C or lower, and is preferably -10°C or lower. Specifically, examples of the component (A) include one or more selected from the group consisting of hydrocarbon oils, volatile silicone oils, and ester oils.

Examples of the hydrocarbon oil include one or more selected from the group consisting of liquid paraffin, squalane, vaseline, and turpentine oil.

Examples of the volatile silicone oil include one or more selected from the group consisting of octamethylcyclotetrasiloxane, decamethylcyclopentasiloxane, dodecamethylcyclohexasiloxane, methylcyclopolysiloxane, octamethyltrisiloxane, tetradecamethylhexasiloxane, low-viscosity methylpolysiloxane, and the like.

Examples of the ester oil include one or more selected from the group consisting of jojoba oil, argan oil, olive oil, coconut oil, and the like.

Among these components (A), from the viewpoint of effectively preventing the undesired generation of the carbon dioxide gas during the manufacturing, during the storage, or before the use, and ensuring excellent storage stability, the hydrocarbon oils are preferred, one or two selected from the group consisting of liquid paraffin and squalane are more preferred, and liquid paraffin is still more preferred.

Examples of the liquid paraffin include a mixture of alkanes having 20 or more carbon atoms, which is a colorless and odorless liquid. Publicly known commercial products of the liquid paraffin can be used without particular limitation, but in the present invention, those which meet standards specified in, for example, the Standards for Quasi-drug Ingredients can be preferably used.

More specifically, as the liquid paraffin used as the component (A), those having a density at 15°C of from 0.800 to 0.900 g/cm³ are preferred, those having the density of from 0.820 to 0.890 g/cm³ are more preferred, those having the density of from 0.845 to 0.885 g/cm³ are still more preferred, and those having the density of from 0.850 to 0.880 g/cm³ are even more preferred. Further, as the liquid paraffin used as the component (A), those having a viscosity at 37.8°C of from 1 to 120 mm²/s are preferred, those having the viscosity of from 10 to 100 mm²/s are more preferred, those having the viscosity of from 37 to 95 mm²/s are still more preferred, those having the viscosity of from 40 to 90 mm²/s are even more preferred, and those having the viscosity of from 74 to 88 mm²/s are further more preferred.

It should be noted that the viscosity of such liquid paraffin is a value measured using a glass capillary viscometer in accordance with the JIS K-2283.

From the viewpoint of effectively enhancing the storage stability and providing the good use impression with the reduced unpleasant oily feeling when the composition is applied to the oral cavity, the content of the component (A) in the nonaqueous composition for oral cavity of the present invention is preferably 15% by mass or more, more preferably 20% by mass or more, still more preferably 25% by mass or more, and even more preferably 30% by mass or more, and is preferably 50% by mass or less, more preferably 45% by mass or less, still more preferably 41% by mass or less, and even more preferably 38% by mass or less. Thus, the content of the component (A) in the nonaqueous composition for oral cavity of the present invention is preferably from 15 to 50% by mass, more preferably from 20 to 45% by mass, still more preferably from 25 to 41% by mass, and even more preferably from 30 to 38% by mass.

The nonaqueous composition for oral cavity of the present invention contains, as the component (B), an acid which is solid at 25°C. When the nonaqueous composition for oral cavity of the present invention is applied to the oral cavity, the presence of the saliva and the like promotes a reaction between the component (B) and the carbonate salt as the component (C) described below, so that bubbles of carbon dioxide gas, which exhibit a characteristic and pleasant fizzy feeling, are generated, thereby providing a refreshing feeling.

Examples of the component (B) include organic acids or amino acids having four or more and six or less carbon atoms and one or more and three or less carboxyl groups in the molecule. More specific examples include, for example, one or more selected from the group consisting of malic acid, citric acid, succinic acid, fumaric acid, tartaric acid, adipic acid, aspartic acid, glutamic acid, and pyroglutamic acid. Among these, from the viewpoint of ensuring the good fizzy feeling, the component (B) is preferably one or more selected from the group consisting of malic acid, citric acid, tartaric acid, and succinic acid, more preferably one or two selected from the group consisting of malic acid and tartaric acid, and still more preferably malic acid.

The content of the malic acid in the component (B) is preferably 20% by mass or more, more preferably 40% by mass or more, still more preferably 60% by mass or more, even more preferably 80% by mass or more, and further more preferably 100% by mass.

The component (B) in the nonaqueous composition for oral cavity of the present invention may or may not have a coating layer formed of a coating material. However, from the viewpoint of generating bubbles of carbon dioxide gas which pleasantly burst in the oral cavity and thereby exhibiting a good fizzy feeling, it is preferable that the component (B) not have the coating layer.

The phrase that the component (B) "does not have a coating layer" means that the coating material does not cover partially or entirely the surface of the component (B), that is, that the coating layer is not formed on a part or the entire of the surface of component (B). The coating material is used as a material for preliminarily coating a part or the entire of the surface of the component (B) in order to suppress undesired reactions in the nonaqueous composition for oral cavity during the manufacturing, during the storage, or before the use and thereby prevent the generation of the carbon dioxide gas in advance. The coating material may not be limited as long as it can coat the part or the entire of the surface of the component (B).

From the viewpoint of generating the bubbles of the carbon dioxide gas which pleasantly burst in the oral cavity and ensuring the good fizzy feeling, the content of the component (B) in the nonaqueous composition for oral cavity of the present invention is preferably 6% by mass or more, more preferably 8% by mass or more, and still more preferably 10% by mass or more, and is preferably 25% by mass or less, more preferably 22% by mass or less, and still more preferably 18% by mass or less. Thus, the content of the component (B) in the nonaqueous composition for oral cavity of the present invention is preferably from 6 to 25% by mass, more preferably from 8 to 22% by mass, and still more preferably from 10 to 18% by mass.

The nonaqueous composition for oral cavity of the present invention contains, as the component (C), a carbonate salt.

Specific examples of the component (C) include, for example, one or more selected from the group consisting of sodium hydrogen carbonate, potassium hydrogen carbonate, calcium hydrogen carbonate, sodium carbonate, potassium carbonate, calcium carbonate, and magnesium carbonate.

Among these, from the viewpoint of enhancing the storage stability, the component (C) is preferably one or two selected from the group consisting of sodium hydrogen carbonate and sodium carbonate, and more preferably contains both sodium hydrogen carbonate and sodium carbonate.

From the viewpoint of enhancing the fizzy feeling, sodium carbonate is preferred.

When the component (C) contains sodium hydrogen carbonate and sodium carbonate, from the viewpoint of generating the bubbles of the carbon dioxide gas which pleasantly burst in the oral cavity and ensuring a good fizzy feeling, the content of the sodium carbonate in 100% by mass of the component (C) is preferably 10% by mass or more, more preferably 15% by mass or more, and is preferably 35% by mass or less, more preferably 30% by mass or less.

In addition, since the component (C) contained in the nonaqueous composition for oral cavity of the present invention has good manufacturing stability and excellent storage stability, like component (B), the component (C) may or may not have a coating layer. However, from the viewpoint of generating the bubbles of the carbon dioxide gas which pleasantly burst in the oral cavity and thereby exhibiting the good fizzy feeling, it is preferable that the component (C) also not have the coating layer.

The phrase that the component (C) "does not have a coating layer" means that the coating material does not cover partially or entirely the surface of the component (C). The coating material is used as a material for preliminarily coating a part or the entire of the surface of the component (C) in order to suppress undesired reactions in the nonaqueous composition for oral cavity during the manufacturing, during the storage, or before the use and thereby prevent the generation of the carbon dioxide gas in advance.

From the viewpoint of generating the bubbles of the carbon dioxide gas which pleasantly burst in the oral cavity and ensuring the good fizzy feeling, the content of the component (C) in the nonaqueous composition for oral cavity of the present invention is preferably 6% by mass or more, more preferably 8% by mass or more, and still more preferably 10% by mass or more, and is preferably 35% by mass or less, more preferably 32% by mass or less, and still more preferably 30% by mass or less. Thus, the content of the component (C) in the nonaqueous composition for oral cavity of the present invention is preferably from 6 to 35% by mass, more preferably from 8 to 32% by mass, and still more preferably from 10 to 30% by mass.

From the viewpoint of generating an appropriate amount of carbon dioxide gas and producing the bubbles which pleasantly burst in the oral cavity, the mass ratio between the content of the component (B) and the content of the component (C) ((B)/(C)) is preferably 0.3 or more, more preferably 0.4 or more, and still more preferably 0.5 or more, and is preferably 1.1 or less, more preferably 1 or less, and still more preferably 0.9 or less. Thus, the mass ratio between the content of the component (B) and the content of the component (C) ((B)/(C)) is preferably from 0.3 to 1.1, more preferably from 0.4 to 1, and still more preferably from 0.5 to 0.9.

From the viewpoint of promoting the dispersion of the bubbles of the carbon dioxide gas in the oral cavity and ensuring the excellent storage stability, the total content of the component (B) and the component (C) in the nonaqueous composition for oral cavity of the present invention is preferably 12% by mass or more, more preferably 15% by mass or more, and still more preferably 18% by mass or more, and is preferably 55% by mass or less, more preferably 53% by mass or less, and still more preferably 50% by mass or less. Thus, the total content of the component (B) and the component (C) in the nonaqueous composition for oral cavity of the present invention is preferably from 12 to 55% by mass, more preferably from 15 to 53% by mass, and still more preferably from 18 to 50% by mass.

From the viewpoint that, when the composition is applied to the oral cavity, the contact with the saliva and the like should promote the reaction between the component (B) and the component (C) to effectively generate the bubbles of the carbon dioxide gas with high diffusion, which provide the good fizzy feeling, thereby exhibiting the good use impression with a reduced unpleasant oily feeling when the composition is applied to the oral cavity, the mass ratio of the total content of the component (B) and the component (C) to the content of the component (A) ({(B) + (C)} / (A)) is preferably 0.2 or more, more preferably 0.3 or more, and still more preferably 0.5 or more, and is preferably 3.5 or less, more preferably 2.5 or less, and still more preferably 2 or less. Thus, the mass ratio of the total content of the component (B) and the component (C) to the content of the component (A) ({(B) + (C)} / (A)) is preferably from 0.2 to 3.5, more preferably from 0.3 to 2.5, and still more preferably from 0.5 to 2.

The nonaqueous composition for oral cavity of the present invention contains, as a component (D), an oil-based gelling agent. The oil-based gelling agent as the component (D) is a component capable of solidifying or gelling the component (A), and also contributes to the exhibition of appropriate shape retention property and ensures the good manufacturing stability.

Examples of the component (D) include fatty acids, fatty acid esters, or condensation products of fatty acids, such as dextrin fatty acid esters, 12-hydroxystearic acid, and oligomers obtained by condensing glycerin and mixed fatty acids composed of behenic acid, and eicosanedioic acid;
petroleum waxes or natural waxes having a melting point (value at 1 atm) of 50°C or higher, such as microcrystalline wax, polyethylene wax, paraffin wax, Fischer-Tropsch wax, carnauba wax, candelilla wax, ozokerite, and ceresin wax;
resin powders such as polyethylene powder;
metal soaps such as zinc stearate, calcium stearate, calcium palmitate, lithium 2-ethylhexanoate, and aluminum 12-hydroxystearate;
N-acyl amino acid amides such as lauroylglutamic acid dibutylamide, lauroylglutamic acid stearyl amide, and dicaproyllysine lauroyl phenylalanine laurylamide; N-acyl amino acid amine salts such as a dicaproyllysine laurylamine salt;
N-acyl amino acid esters such as a dicaproyllysine lauryl ester;
and one or more selected from the group consisting of clay minerals such as bentonite, organically modified bentonite, and laponite.

Among these, from the viewpoint of effectively promoting the solidification or the gelation of the component (A) and effectively suppressing unnecessary reactions between the components (B) and (C), the components (D) is preferably one or more selected from the group consisting of fatty acids, fatty acid esters, or condensation products of fatty acids, and is more preferably a dextrin fatty acid ester.

From the viewpoint of satisfactorily solidifying or gelling the component (A) and preventing the generation of the carbon dioxide gas in advance, the content of the component (D) in the nonaqueous composition for oral cavity of the present invention is preferably 0.5% by mass or more, more preferably 0.7% by mass or more, and still more preferably 1% by mass or more, and is preferably 5% by mass or less, more preferably 4% by mass or less, still more preferably 3% by mass or less, even more preferably 2.3% by mass or less, and further more preferably 1.8% by mass or less. Thus, the content of the component (D) in the nonaqueous composition for oral cavity of the present invention is preferably from 0.5 to 5% by mass, more preferably from 0.7 to 4% by mass, still more preferably from 1 to 3% by mass, even more preferably from 1 to 2.3% by mass, and further more preferably from 1 to 1.8% by mass.

From the viewpoint that, when the composition is applied to the oral cavity, the components (B) and (C) should generate the bubbles of the carbon dioxide gas which pleasantly burst in the oral cavity, thereby ensuring the good fizzy feeling and providing the good use impression with the suppressed unpleasant oily feeling, the mass ratio between the content of the component (A) and the content of the component (D) ((A)/(D)) is preferably 10 or more, more preferably 12 or more, still more preferably 15 or more, even more preferably 18 or more, and further more preferably 20.5 or more, and is preferably 30 or less, more preferably 27 or less, and still more preferably 25 or less. Thus, the mass ratio between the content of the component (A) and the content of the component (D) ((A)/(D)) is preferably from 10 to 30, more preferably from 12 to 27, still more preferably from 15 to 25, even more preferably from 18 to 25, and further preferably from 20.5 to 25.

The nonaqueous composition for oral cavity of the present invention contains, as a component (E), a sugar alcohol which is solid at 25°C. From the viewpoints of ensuring the good fizzy feeling together with the good use impression with the suppressed unpleasant oily feeling when the composition is applied to the oral cavity, and enabling rapid dissolution and diffusion by the saliva upon application to the oral cavity, the solubility of the component (E) in water at 25°C (an amount dissolved in 100% by mass of a saturated aqueous solution at 25°C) is preferably 5% by mass or more, more preferably 8% by mass or more, still more preferably 12% by mass or more, and even more preferably 20% by mass or more, and is preferably 75% by mass or less, more preferably 65% by mass or less, and still more preferably 55% by mass or less. Thus, the solubility of the component (E) in water at 25°C is preferably from 5 to 75% by mass, preferably from 8 to 65% by mass, more preferably from 12 to 55% by mass, and even more preferably from 20 to 55% by mass.

Specific examples of the component (E) include, for example, one or more selected from the group consisting of erythritol, mannitol, sorbitol, xylitol, and reduced palatinose (a mixture of α-D-glucopyranosyl-1,6-sorbitol and α-D-glucopyranosyl-1,6-mannitol).

Among these, from the viewpoint of ensuring the good use impression with the suppressed oily feeling, the component (E) is preferably one or more selected from the group consisting of erythritol, xylitol, and mannitol, more preferably one or two selected from erythritol and xylitol, and still more preferably erythritol.

From the viewpoint of ensuring the good fizzy feeling together with the good use impression with the suppressed unpleasant oily feeling when the composition is applied to the oral cavity the content of the component (E) in the nonaqueous composition for oral cavity of the present invention is preferably 2% by mass or more, more preferably 5% by mass or more, still more preferably 8% by mass or more, and even more preferably 15% by mass or more, and is preferably 45% by mass or less, more preferably 40% by mass or less, and still more preferably 35% by mass or less. Thus, the content of the component (E) in the nonaqueous composition for oral cavity of the present invention is preferably from 2 to 45% by mass, more preferably from 5 to 45% by mass, still more preferably from 8 to 40% by mass, and even more preferably from 15 to 35% by mass.

From the viewpoint of ensuring both the good use impression with the suppressed unpleasant oily feeling and the good fizzy feeling when the composition is applied to the oral cavity, the mass ratio between the content of the component (A) and the content of the component (E) ((A)/(E)) is preferably 0.4 or more, more preferably 0.6 or more, more preferably 0.8 or more, and still more preferably 1 or more, and is preferably 15 or less, more preferably 8 or less, still more preferably 4 or less, and even more preferably 3.5 or less. Thus, the mass ratio between the content of the component (A) and the content of the component (E) ((A)/(E)) is preferably from 0.4 to 15, more preferably from 0.6 to 8, still more preferably from 0.8 to 4, and even more preferably from 1 to 3.5.

From the viewpoint of ensuring both the good use impression with the suppressed unpleasant oily feeling and the good fizzy feeling when the composition is applied to the oral cavity, the mass ratio between the content of the component (B) and the content of the component (E) ((B)/(E)) is preferably 0.1 or more, more preferably 0.2 or more, still more preferably 0.3 or more, and even more preferably 0.5 or more, and is preferably 5 or less, more preferably 2 or less, still more preferably 1.5 or less, and even more preferably 1 or less. Thus, the mass ratio between the content of the component (B) and the content of the component (E) ((B)/(E)) is preferably from 0.1 to 5, more preferably from 0.2 to 2, still more preferably from 0.3 to 1.5, and even more preferably from 0.5 to 1.

From the viewpoint of ensuring both the good use impression with the suppressed unpleasant oily feeling and the good fizzy feeling when the composition is applied to the oral cavity, the mass ratio between the content of the component (C) and the content of the component (E) ((C)/(E)) is preferably 0.1 or more, more preferably 0.2 or more, still more preferably 0.3 or more, and even more preferably 0.35 or more, and is preferably 8 or less, more preferably 3 or less, still more preferably 2.5 or less, and even more preferably 1.5 or less. Thus, the mass ratio between the content of the component (C) and the content of the component (E) ((C)/(E)) is preferably from 0.1 to 8, more preferably from 0.2 to 3, still more preferably from 0.3 to 2.5, and even more preferably from 0.35 to 1.5.

From the viewpoint of effectively suppressing unnecessary reactions between the components (B) and (C) in the nonaqueous composition for oral cavity during the manufacturing, during the storage, or before the use, preventing the generation of the carbon dioxide gas in advance, and thereby ensuring the good manufacturing stability and the excellent storage stability, it is also preferred that the nonaqueous composition for oral cavity of the present invention further contains one or more metal compounds (F) selected from the group consisting of zinc oxide, magnesium oxide, calcium oxide, aluminum oxide, titanium oxide, sodium sulfate, magnesium sulfate, calcium sulfate, calcium chloride, magnesium chloride, zinc chloride, magnesium silicate, and calcium silicate. Among these, from the viewpoint of effectively preventing the generation of the carbon dioxide gas, the component (F) is preferably one or more selected from the group consisting of zinc oxide, magnesium oxide, and magnesium sulfate, and more preferably one or two selected from the group consisting of zinc oxide and magnesium oxide.

From the viewpoint of effectively preventing the generation of the carbon dioxide gas in the nonaqueous composition for oral cavity during the storage or before the use, the content of the component (F) is, preferably 0.5% by mass or more, more preferably 0.7% by mass or more, and still more preferably 0.8% by mass or more, and is preferably 7% by mass or less, more preferably 5% by mass or less, and still more preferably 3% by mass or less. Thus, the content of the component (F) in the nonaqueous composition for oral cavity of the present invention is preferably from 0.5 to 7% by mass, more preferably from 0.7 to 5% by mass, and still more preferably from 0.8 to 3% by mass.

From the viewpoint of effectively preventing the generation of the carbon dioxide gas in the nonaqueous composition for oral cavity during the storage or before the use, the mass ratio between the content of the component (F) and the content of the component (A) ((F)/(A)) is preferably 0.005 or more, more preferably 0.008 or more, and still more preferably 0.01 or more, and is preferably 0.1 or less, more preferably 0.08 or less, and still more preferably 0.05 or less. Thus, the mass ratio between the content of the component (F) and the content of the component (A) ((F)/(A)) is preferably from 0.005 to 0.1, more preferably from 0.008 to 0.08, and even more preferably from 0.01 to 0.05.

From the viewpoint of effectively preventing the generation of the carbon dioxide gas in the nonaqueous composition for oral cavity during the storage or before the use, the mass ratio between the content of the component (F) and the content of the component (B) ((F)/(B)) is preferably 0.01 or more, more preferably 0.03 or more, and still more preferably 0.05 or more, and is preferably 0.7 or less, more preferably 0.6 or less, and still more preferably 0.5 or less. Thus, the mass ratio between the content of the component (F) and the content of the component (B) ((F)/(B)) is preferably from 0.01 to 0.7, more preferably from 0.03 to 0.6, and still more preferably from 0.05 to 0.5.

From the viewpoint of effectively preventing the generation of the carbon dioxide gas in the nonaqueous composition for oral cavity during the storage or before the use, the mass ratio between the content of the component (F) and the content of the component (C) ((F)/(C)) is preferably 0.005 or more, more preferably 0.008 or more, and still more preferably 0.01 or more, and is preferably 0.3 or less, more preferably 0.2 or less, and still more preferably 0.1 or less. Thus, the mass ratio between the content of the component (F) and the content of the component (C) ((F)/(C)) is preferably from 0.005 to 0.3, more preferably from 0.008 to 0.2, and still more preferably from 0.01 to 0.1.

From the viewpoint of increasing abrasiveness, it is preferable that the nonaqueous composition for oral cavity of the present invention further contain anhydrous silica. Examples of the anhydrous silica include fumed, porous, nonporous, or spherical abrasive silicas and thickening silicas, or anhydrous silicas obtained by subjecting these to a hydrophobization treatment.

From the viewpoint of effectively increasing the abrasiveness and effectively preventing the generation of the carbon dioxide gas in the nonaqueous composition for oral cavity during the storage or before the use, the content of the anhydrous silica in the nonaqueous composition for oral cavity of the present invention is preferably 0.1% by mass or more, more preferably 0.5% by mass or more, and still more preferably 1% by mass or more, and is preferably 6.9% by mass or less, more preferably 6.5% by mass or less, and still more preferably 6% by mass or less. Thus, the content of the anhydrous silica in the nonaqueous composition for oral cavity of the present invention is preferably from 0.1 to 6.9% by mass, more preferably from 0.5 to 6.5% by mass, and still more preferably from 1 to 6% by mass.

On the other hand, from the viewpoint of effectively suppressing unnecessary reactions between the components (B) and (C) in the nonaqueous composition for oral cavity of the present invention during the manufacturing, during the storage, or before the use to prevent the generation of the carbon dioxide gas in advance and thereby ensuring the good manufacturing stability and the excellent storage stability, it is preferable to limit the content of the anhydrous silica.

The content of the anhydrous silica in the nonaqueous composition for oral cavity of the present invention is preferably less than 10% by mass, more preferably less than 7% by mass, still more preferably less than 5% by mass, even more preferably less than 4% by mass, and further more preferably less than 1% by mass. Alternatively, it is preferable that the nonaqueous composition for oral cavity of the present invention substantially not contain anhydrous silica. It should be noted that the phrase "substantially does not contain anhydrous silica" means that the content of the anhydrous silica in the composition for oral cavity is less than 0.1% by mass.

From the viewpoint of effectively suppressing unnecessary reactions between the components (B) and (C) in the nonaqueous composition for oral cavity of the present invention during the manufacturing, during the storage, or before the use to prevent the generation of the carbon dioxide gas in advance and thereby ensuring the good manufacturing stability and the excellent storage stability, and securing the good fizzy feeling, it is preferable to limit the content of the nonaqueous solvent having a relative permittivity at 25°C of 10 or more. Examples of the nonaqueous solvent having a relative permittivity at 25°C of 10 or more include, for example, glycerin, polyethylene glycol, 1,3-butylene glycol, propylene glycol, dipropylene glycol, and diglycerin. The content of the nonaqueous solvent having a relative permittivity at 25°C of 10 or more is preferably less than 18% by mass, more preferably 10% by mass or less, still more preferably 6% by mass or less, and even more preferably 3% by mass or less. Alternatively, it is preferable that the nonaqueous composition for oral cavity of the present invention substantially not contain a nonaqueous solvent having a relative permittivity at 25°C of 10 or more. It should be noted that the phrase "substantially does not contain a nonaqueous solvent having a relative permittivity at 25°C of 10 or more" means that the content of the nonaqueous solvent having a relative permittivity at 25°C of 10 or more in the composition for oral cavity is less than 1% by mass.

The nonaqueous composition for oral cavity of the present invention may contain, as components other than the components described above, antibacterial agents or germicides, pharmacologically active ingredients, flavors, colorants, and the like, as long as such components do not impair the effects of the present invention.

The nonaqueous composition for oral cavity of the present invention is filled into a deformable container. By applying an external force or the like to the container, the content, that is, the nonaqueous composition for oral cavity, is discharged through a discharge port provided in the container and then is used.

The container may not be limited in terms of the form, as long as it is provided with the discharge port. Examples of the container form include a tube container provided with a container body and a cap portion having a discharge port, a spout container provided with a pouch container body, and a squeeze-type container provided with a squeezably deformable container body. Further, the material of the container is also not particularly limited. For example, in the case of a tube container provided with a container body having a multilayer structure as a barrel portion, the multilayer structure may include, in addition to an innermost layer made of polyethylene, such as low-density polyethylene, a metal layer made of aluminum or the like and a synthetic resin layer made of polypropylene, polyethylene terephthalate, or the like.

According to the nonaqueous composition for oral cavity of the present invention, it is possible to effectively prevent excessive swelling of the container during the storage, because the composition exhibits excellent storage stability regardless of the type of container in which it is filled.

With regard to the embodiments described above, the present invention further discloses the following nonaqueous composition for oral cavity.
[1] A nonaqueous composition for oral cavity, comprising the following components (A) to (E):
   (A) a nonaqueous solvent having a relative permittivity at 25°C of less than 10 and a melting point of 0°C or lower;
   (B) an acid which is solid at 25°C;
   (C) a carbonate salt;
   (D) an oil-based gelling agent; and
   (E) a sugar alcohol which is solid at 25°C.
[2] The nonaqueous composition for oral cavity according to [1], wherein the relative permittivity at 25°C of the component (A) is preferably 5 or less, and more preferably 3 or less, and the melting point of the component (A) is 0°C or lower, preferably -10°C or lower.
[3] The nonaqueous composition for oral cavity according to [1] or [2], wherein the component (A) is one or more selected from the group consisting of hydrocarbon oils, volatile silicone oils, and ester oils, preferably a hydrocarbon oil, more preferably one or two selected from the group consisting of liquid paraffin and squalane, and still more preferably liquid paraffin.
[4] The nonaqueous composition for oral cavity according to [3], wherein the liquid paraffin preferably has a density at 15°C of from 0.800 to 0.900 g/cm³, more preferably from 0.820 to 0.890 g/cm³, still more preferably from 0.845 to 0.885 g/cm³, and even more preferably from 0.860 to 0.880 g/cm³, and preferably has viscosity at 37.8°C of from 1 to 120 mm²/s, more preferably from 10 to 100 mm²/s, still more preferably from 37 to 95 mm²/s, even more preferably from 40 to 90 mm²/s, and further preferably from 74 to 88 mm²/s.
[5] The nonaqueous composition for oral cavity according to any one of [1] to [4], wherein the content of the component (A) is preferably 15% by mass or more, more preferably 20% by mass or more, still more preferably 25% by mass or more, and even more preferably 30% by mass or more, and is preferably 50% by mass or less, more preferably 45% by mass or less, still more preferably 41% by mass or less, and even more preferably 38% by mass or less.
[6] The nonaqueous composition for oral cavity according to any one of [1] to [5], wherein the component (B) is an organic acids or an amino acid having four or more and six or less carbon atoms and one or more and three or less carboxyl groups in the molecule, which is more preferably one or more selected from the group consisting of malic acid, citric acid, succinic acid, fumaric acid, tartaric acid, adipic acid, aspartic acid, glutamic acid, and pyroglutamic acid, still more preferably one or more selected from the group consisting of malic acid, citric acid, tartaric acid, and succinic acid, still more preferably one or two selected from the group consisting of malic acid and tartaric acid, and even more preferably malic acid.
[7] The nonaqueous composition for oral cavity according to any one of [1] to [6], wherein the content of the component (B) is preferably 6% by mass or more, more preferably 8% by mass or more, still more preferably 10% by mass or more, and is preferably 25% by mass or less, more preferably 22% by mass or less, and still more preferably 18% by mass or less.
[8] The nonaqueous composition for oral cavity according to any one of [1] to [7], wherein the component (C) is preferably one or more selected from the group consisting of sodium hydrogen carbonate, potassium hydrogen carbonate, calcium hydrogen carbonate, sodium carbonate, potassium carbonate, calcium carbonate, and magnesium carbonate, more preferably one or two selected from the group consisting of sodium hydrogen carbonate and sodium carbonate, and still more preferably contains both sodium hydrogen carbonate and sodium carbonate.
[9] The nonaqueous composition for oral cavity according to [8], wherein the content of the sodium carbonate in 100% by mass of the component (C) is preferably 10% by mass or more, more preferably 15% by mass or more, and preferably 35% by mass or less, more preferably 30% by mass or less.
[10] The nonaqueous composition for oral cavity according to any one of [1] to [9], wherein the content of the component (C) is preferably 6% by mass or more, more preferably 8% by mass or more, and still more preferably 10% by mass or more, and is preferably 35% by mass or less, more preferably 32% by mass or less, and still more preferably 30% by mass or less.
[11] The nonaqueous composition for oral cavity according to any one of [1] to [10], wherein the mass ratio between the content of the component (B) and the content of the component (C) ((B)/(C)) is preferably 0.3 or more, more preferably 0.4 or more, and still more preferably 0.5 or more, and is preferably 1.1 or less, more preferably 1 or less, and still more preferably 0.9 or less.
[12] The nonaqueous composition for oral cavity according to any one of [1] to [11], wherein the mass ratio of the total content of the component (B) and the component (C) to the content of the component (A) ({(B) + (C)} / (A)) is preferably 0.2 or more, more preferably 0.3 or more, and still more preferably 0.5 or more, and is preferably 3.5 or less, more preferably 2.5 or less, and still more preferably 2 or less.
[13] The nonaqueous composition for oral cavity according to any one of [1] to [12], wherein the component (D) is preferably one or more selected from the group consisting of fatty acids, fatty acid esters, and condensation products of fatty acids, and more preferably a dextrin fatty acid ester.
[14] The nonaqueous composition for oral cavity according to any one of [1] to [13], wherein the content of the component (D) is preferably 0.5% by mass or more, more preferably 0.7% by mass or more, and still more preferably 1% by mass or more, and is preferably 5% by mass or less, more preferably 4% by mass or less, still more preferably 3% by mass or less, even more preferably 2.3% by mass or less, and further more preferably 1.8% by mass or less.
[15] The nonaqueous composition for oral cavity according to any one of [1] to [14], wherein the mass ratio between the content of the component (A) and the content of the component (D) ((A)/(D)) is preferably 10 or more, more preferably 12 or more, still more preferably 15 or more, even more preferably 18 or more, and further more preferably 20.5 or more, and is preferably 30 or less, more preferably 27 or less, and still more preferably 25 or less.
[16] The nonaqueous composition for oral cavity according to any one of [1] to [15], wherein the water solubility of the component (E) at 25°C (an amount dissolved in 100% by mass of a saturated aqueous solution at 25°C) is preferably 5% by mass or more, more preferably 8% by mass or more, still more preferably 12% by mass or more, and even more preferably 20% by mass or more, and is preferably 75% by mass or less, more preferably 65% by mass or less, and even more preferably 55% by mass or less.
[17] The nonaqueous composition for oral cavity according to any one of [1] to [16], wherein the component (E) is one or more selected from the group consisting of erythritol, mannitol, sorbitol, xylitol, and reduced palatinose (a mixture of α-D-glucopyranosyl-1,6-sorbitol and α-D-glucopyranosyl-1,6-mannitol), more preferably one or more selected from erythritol, xylitol, and mannitol, still more preferably one or two selected from the group consisting of erythritol and xylitol, and even more preferably erythritol.
[18] The nonaqueous composition for oral cavity according to any one of [1] to [17], wherein the content of the component (E) is preferably 2% by mass or more, more preferably 5% by mass or more, still more preferably 8% by mass or more, and even more preferably 15% by mass or more, and is preferably 45% by mass or less, more preferably 40% by mass or less, and still more preferably 35% by mass or less.
[19] The nonaqueous composition for oral cavity according to any one of [1] to [18], wherein the mass ratio between the content of the component (A) and the content of the component (E) ((A)/(E)) is preferably 0.4 or more, more preferably 0.6 or more, still more preferably 0.8 or more, and even more preferably 1 or more, and is preferably 15 or less, more preferably 8 or less, still more preferably 4 or less, and even more preferably 3.5 or less.
[20] The nonaqueous composition for oral cavity according to any one of [1] to [19], wherein the mass ratio between the content of the component (B) and the content of the component (E) ((B)/(E)) is preferably 0.1 or more, more preferably 0.2 or more, still more preferably 0.3 or more, and even more preferably 0.5 or more, and is preferably 5 or less, more preferably 2 or less, still more preferably 1.5 or less, and even more preferably 1 or less.
[21] The nonaqueous composition for oral cavity according to any one of [1] to [20], wherein the mass ratio between the content of the component (C) and the content of the component (E) ((C)/(E)) is preferably 0.1 or more, more preferably 0.2 or more, still more preferably 0.3 or more, and even more preferably 0.35 or more, and is preferably 8 or less, more preferably 3 or less, still more preferably 2.5 or less, and even more preferably 1.5 or less.
[22] The nonaqueous composition for oral cavity according to any one of [1] to [21], further comprising one or more metal compounds (F) selected from the group consisting of zinc oxide, magnesium oxide, calcium oxide, aluminum oxide, titanium oxide, sodium sulfate, magnesium sulfate, calcium sulfate, calcium chloride, magnesium chloride, zinc chloride, magnesium silicate, and calcium silicate.
[23] The nonaqueous composition for oral cavity according to [22], wherein the component (F) is preferably one or more selected from the group consisting of zinc oxide, magnesium oxide, and magnesium sulfate, more preferably one or two selected from the group consisting of zinc oxide and magnesium oxide.
[24] The nonaqueous composition for oral cavity according to [22] or [23], wherein the content of the component (F) is preferably 0.5% by mass or more, more preferably 0.7% by mass or more, and still more preferably 0.8% by mass or more, and is preferably 7% by mass or less, more preferably 5% by mass or less, and still more preferably 3% by mass or less.
[25] The nonaqueous composition for oral cavity according to any one of [22] to [24], wherein the mass ratio between the content of the component (F) and the content of the component (A) ((F)/(A)) is preferably 0.005 or more, more preferably 0.008 or more, and still more preferably 0.01 or more, and is preferably 0.1 or less, more preferably 0.08 or less, and still more preferably 0.05 or less.
[26] The nonaqueous composition for oral cavity according to any one of [22] to [25], wherein the mass ratio between the content of the component (F) and the content of the component (B) ((F)/(B)) is preferably 0.01 or more, more preferably 0.03 or more, and still more preferably 0.05 or more, and is preferably 0.7 or less, more preferably 0.6 or less, and still more preferably 0.5 or less.
[27] The nonaqueous composition for oral cavity according to any one of [22] to [26], wherein the mass ratio between the content of the component (F) and the content of the component (C) ((F)/(C)) is preferably 0.005 or more, more preferably 0.008 or more, and still more preferably 0.01 or more, and is preferably 0.3 or less, more preferably 0.2 or less, and still more preferably 0.1 or less.
[28] The nonaqueous composition for oral cavity according to any one of [1] to [27], wherein a content of anhydrous silica is preferably 0.1% by mass or more, more preferably 0.5% by mass or more, and still more preferably 1% by mass or more, and is preferably 6.9% by mass or less, more preferably 6.5% by mass or less, and still more preferably 6% by mass or less, or preferably less than 10% by mass, more preferably less than 7% by mass, still more preferably less than 5% by mass, even more preferably less than 4% by mass, and further more preferably less than 1% by mass, or, preferably the nonaqueous composition for oral cavity of the present invention substantially does not contain anhydrous silica.
[29] The nonaqueous composition for oral cavity according to any one of [1] to [28], wherein the content of the nonaqueous solvent having a relative permittivity at 25°C of 10 or more is preferably less than 18% by mass, more preferably 10% by mass or less, still more preferably 6% by mass or less, and even more preferably 3% by mass or less, or, preferably the nonaqueous composition for oral cavity substantially of the present invention substantially does not contain the nonaqueous solvent having a relative permittivity at 25°C of 10 or more.
[30] The nonaqueous composition for oral cavity according to any one of [1] to [29], wherein the component (B) and/or the component (C) preferably has no coating layer.

### Examples

Hereinafter, the present invention will be described specifically based on Examples. Unless otherwise indicated in the table, the content of each component is shown in % by mass.

### [Examples 1 to 11 and Comparative Examples 1 to 5]

Respective nonaqueous composition for oral cavity were produced by a conventional method in accordance with the formulations shown in Table 1, and first, they were evaluated for the manufacturing stability in accordance with the method described below. For those which were evaluated as Z1 to Z3, further evaluations were omitted as they were determined to have problems as products. Next, each resulting nonaqueous composition for oral cavity was filled into a tube container (manufactured by Kyodo Printing Co., 32 ϕ aluminum/polyethylene tube, 100 g capacity), after which each evaluation was performed in accordance with the following methods.

The results are shown in the Table 1.

### [Evaluation of Manufacturing Stability]

When each of the nonaqueous composition for oral cavity was produced, its stability was evaluated in accordance with the following criteria:
X: The composition was able to be manufactured without problems.
Z1: The composition had poor dispersion of components and lacked shape retention property.
Z2: Components reacted during the manufacture, causing generation of bubbles.
Z3: Partial liquefaction occurred, resulting in phase separation.

### [Evaluation of Fizzy Feeling]

One gram of each nonaqueous composition for oral cavity was dispensed from its tube container onto a toothbrush, and brushing was performed for two minutes. The fizzy feeling during the brushing was evaluated in accordance with the following criteria:
5: Very pleasant burst of bubbles was felt.
4: Pleasant burst of bubbles was felt.
3: Slight burst of bubbles was felt.
2: Bubbles were perceptible, but no burst feeling was obtained.
1: No bubbles were perceptible.

### [Evaluation of Oily Feeling]

One gram of each nonaqueous composition for oral cavity was dispensed from its tube container onto a toothbrush, and brushing was performed for two minutes. The oily feeling during the brushing was evaluated in accordance with the following criteria:
5: Not oily at all.
4: Not very oily.
3: Somewhat oily but acceptable.
2: Oily but acceptable.
1: Very oily.

### [Evaluation of Refreshing Feeling]

One gram of each nonaqueous composition for oral cavity was dispensed from its tube container onto a toothbrush, and brushing was performed for two minutes. The refreshing feeling felt in the oral cavity after the brushing was evaluated in accordance with the following criteria, using the degree of freshness as an indicator:
5: Very fresh feeling was produced in the mouth.
4: Fresh feeling was produced in the mouth.
3: Slightly fresh feeling was produced in the mouth.
2: Not very fresh feeling was produced, but the feeling was acceptable.
1: No fresh feeling was produced in the mouth.

### [Evaluation of Storage Stability]

Each nonaqueous composition for oral cavity was filled into a tube container at room temperature (25°C), and then was evaluated for the degree of container swelling by visual inspection after the storage in an incubator set at 50°C for the specified period as described below, in accordance with the following criteria.

It should be noted that Figure 1 illustrates photographs of the containers after the storage for the Example 1, the Comparative Example 1 (swelling was confirmed ten minutes after the filling), and the Comparative Example 2 (swelling was confirmed one hour after the filling).

The criteria are as follows:
5: No swelling was observed for one month of storage.
4: No swelling was observed for two weeks of storage.
3: No swelling was observed for one week of storage.
2: Swelling was observed within one week of storage.
1: Swelling was observed on the same day of the filling into the tube container, even before the storage in the incubator.

**[Table 1]**

| | | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 | Comparative Example 4 | Comparative Example 5 |
|---|---|---|---|---|---|---|---|---|---|---|---|---|---|---|---|---|---|
| (A) | Liquid paraffin A^{*1} | 31.50 | 31.50 | 31.50 | 31.50 | 36.40 | 31.50 | 42.60 | 36.40 | 40.00 | 46.00 | | | | 31.50 | 31.50 | 42.60 |
| | Liquid paraffin B^{*2} | | | | | | | | | | | 31.50 | | | | | |
| | Glycerin | | | | | | | | | | | | | 31.50 | | | |
| | 1,3-butylene glycol | | | | | | | | | | | | 31.50 | | | | |
| (D) | Dextrin palmitate | 1.50 | 1.50 | 1.50 | 1.50 | 1.60 | 1.50 | 2.40 | 1.60 | 2.00 | 5.00 | 1.50 | 1.50 | | 1.50 | 1.50 | |
| | Sodium carboxymethyl cellulose | | | | | | | | | | | | | 1.50 | | | 2.40 |
| (B) | Malic acid | 16.00 | 16.00 | 8.00 | | 12.00 | 16.00 | 16.00 | 16.00 | 16.00 | 16.00 | 16.00 | 16.00 | 16.00 | | 16.00 | 16.00 |
| | Tartaric acid | | | 8.00 | 16.00 | | | | | | | | | | | | |
| | Lactic acid | | | | | | | | | | | | | | 16.00 | | |
| (C) | Sodium hydrogen carbonate | 20.00 | 20.00 | 20.00 | 20.00 | 10.00 | 20.00 | 20.00 | 20.00 | 20.00 | 20.00 | 20.00 | 20.00 | 20.00 | 20.00 | | 20.00 |
| | Sodium carbonate | 8.00 | 8.00 | 8.00 | 8.00 | 4.00 | 8.00 | 8.00 | 4.00 | | 8.00 | 8.00 | 8.00 | 8.00 | 8.00 | | 8.00 |
| | Disodium hydrogen phosphate | | | | | | | | | | | | | | | 28.00 | |
| (E) | Erythritol | 22.00 | | 22.00 | 22.00 | 32.00 | 22.00 | 10.00 | 22.00 | 22.00 | 4.00 | 22.00 | 22.00 | 22.00 | 22.00 | 22.00 | 10.00 |
| | Xylitol | | 22.00 | | | | | | | | | | | | | | |
| | Anhydrous silica | | | | | 3.00 | | | | | | | | | | | |
| (F) | Zinc oxide | 1.00 | 1.00 | 1.00 | 1.00 | 1.00 | | 1.00 | | | 1.00 | 1.00 | 1.00 | 1.00 | 1.00 | 1.00 | 1.00 |
| | Magnesium oxide | | | | | | 1.00 | | | | | | | | | | |
| | Total | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| | (A)/(E) | 1.43 | 1.43 | 1.43 | 1.43 | 1.14 | 1.43 | 4.26 | 1.65 | 1.82 | 11.50 | 1.43 | 0 | 0 | 1.43 | 1.43 | 4.26 |
| | (B)/(E) | 0.73 | 0.73 | 0.73 | 0.73 | 0.38 | 0.73 | 1.60 | 0.73 | 0.73 | 4.00 | 0.73 | 0.73 | 0.73 | 0.00 | 0.73 | 1.60 |
| | (C)/(E) | 1.27 | 1.27 | 1.27 | 1.27 | 0.44 | 1.27 | 2.80 | 1.09 | 0.91 | 7.00 | 1.27 | 1.27 | 1.27 | 1.27 | 0 | 2.80 |
| | (A)/(D) | 21.00 | 21.00 | 21.00 | 21.00 | 22.75 | 21.00 | 17.75 | 22.75 | 20.00 | 9.20 | 21.00 | 0.00 | - | 21.00 | 21.00 | - |
| | (B)+(C) | 44.00 | 44.00 | 44.00 | 44.00 | 26.00 | 44.00 | 44.00 | 40.00 | 36.00 | 44.00 | 44.00 | 44.00 | 44.00 | 28.00 | 16.00 | 44.00 |
| | {(B)+(C)}/(A) | 1.40 | 1.40 | 1.40 | 1.40 | 0.71 | 1.40 | 1.03 | 1.10 | 0.90 | 0.96 | 1.40 | - | - | 0.89 | 0.51 | 1.03 |
| | (B)/(C) | 0.57 | 0.57 | 0.57 | 0.57 | 0.86 | 0.57 | 0.57 | 0.67 | 0.80 | 0.57 | 0.57 | 0.57 | 0.57 | 0.00 | - | 0.57 |
| | (F)/(A) | 0.03 | 0.03 | 0.03 | 0.03 | 0.03 | 0.03 | 0.02 | 0.00 | 0.00 | 0.02 | 0.03 | - | - | 0.03 | 0.03 | 0.02 |
| | (F)/(B) | 0.06 | 0.06 | 0.06 | 0.06 | 0.08 | 0.06 | 0.06 | 0.00 | 0.00 | 0.06 | 0.06 | 0.06 | 0.06 | - | 0.06 | 0.06 |
| | (F)/(C) | 0.04 | 0.04 | 0.04 | 0.04 | 0.07 | 0.04 | 0.04 | 0.00 | 0.00 | 0.04 | 0.04 | 0.04 | 0.04 | 0.04 | - | 0.04 |
| Manufacturing stability | | X | X | X | X | X | X | X | X | X | X | X | Z1 | X | Z2 | X | Z3 |
| Fizzy feeling | | 4 | 4 | 4 | 3 | 4 | 4 | 4 | 4 | 5 | 4 | 5 | | 3 | | 1 | |
| Oily feeling | | 4 | 4 | 4 | 4 | 5 | 4 | 3 | 4 | 4 | 2 | 2 | | 5 | | 3 | |
| Refreshing feeling | | 5 | 5 | 5 | 5 | 5 | 5 | 4 | 5 | 5 | 2 | 2 | - | 3 | | 2 | |
| Storage stability | | 5 | 5 | 5 | 5 | 5 | 5 | 5 | 5 | 3 | 5 | 5 | 1 | 1 | | 5 | |

| | | | | | | | | | | | | | | | | | |
|---|---|---|---|---|---|---|---|---|---|---|---|---|---|---|---|---|---|
| *1:HICALL K-350 (available from Kaneda), density at 15°C:0.867g/cm³, viscosity at 37.8°C:81.2mm²/s *2:HICALL K-160 (available from Kaneda), density at 15°C:0.839g/cm³, viscosity at 37.8°C:7.35mm²/s | | | | | | | | | | | | | | | | | |

## Claims

1. A nonaqueous composition for oral cavity, comprising the following components (A) to (E):
(A) a nonaqueous solvent having a relative permittivity at 25°C of less than 10 and a melting point of 0°C or lower;
(B) an acid which is solid at 25°C;
(C) a carbonate salt;
(D) an oil-based gelling agent; and
(E) a sugar alcohol which is solid at 25°C.

2. The nonaqueous composition for oral cavity according to Claim 1, wherein solubility of the component (E) in water at 25°C is 5% by mass or more and 75% by mass or less.

3. The nonaqueous composition for oral cavity according to Claim 1 or 2, wherein a mass ratio between a content of the component (A) and a content of the component (E) ((A)/(E)) is 0.4 or more and 5 or less.

4. The nonaqueous composition for oral cavity according to any one of Claims 1 to 3, wherein a mass ratio between the content of the component (A) and a content of the component (D) ((A)/(D)) is 10 or more and 30 or less.

5. The nonaqueous composition for oral cavity according to any one of Claims 1 to 4, wherein a mass ratio of a total content of the component (B) and the component (C) to the content of the component (A) ({(B) + (C)}/ (A)) is 0.2 or more and 3.5 or less.

6. The nonaqueous composition for oral cavity according to any one of Claims 1 to 5, wherein the component (C) comprises sodium hydrogen carbonate and sodium carbonate.

7. The nonaqueous composition for oral cavity according to any one of Claims 1 to 6, wherein the content of the component (B) is 6% by mass or more and 25% by mass or less.

8. The nonaqueous composition for oral cavity according to any one of Claims 1 to 7, wherein a content of anhydrous silica is less than 7% by mass.

9. The nonaqueous composition for oral cavity according to any one of Claims 1 to 8, wherein a content of a nonaqueous solvent having a relative permittivity at 25°C of 10 or more is less than 18% by mass.

10. The nonaqueous composition for oral cavity according to any one of Claims 1 to 9, further comprising one or more metal compounds (F) selected from the group consisting of zinc oxide, magnesium oxide, calcium oxide, aluminum oxide, titanium oxide, sodium sulfate, magnesium sulfate, calcium sulfate, calcium chloride, magnesium chloride, zinc chloride, magnesium silicate, and calcium silicate.
